(19) Europäisches Patentamt
European Patent Office
Office européen des brevets

(11) EP 4 108 230 A1

(12) EUROPEAN PATENT APPLICATION

(43) Date of publication:
**28.12.2022 Bulletin 2022/52**

(21) Application number: **21181419.9**

(22) Date of filing: **24.06.2021**

(51) International Patent Classification (IPC):
***A61K 9/00*** *(2006.01)* ***A61K 31/439*** *(2006.01)*
***A61P 11/00*** *(2006.01)* ***A61K 9/16*** *(2006.01)*

(52) Cooperative Patent Classification (CPC):
**A61K 9/0075; A61K 9/1623; A61K 31/439; A61P 11/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Laboratoires SMB**
**1080 Bruxelles (BE)**

(72) Inventors:
- **BAUDIER, Philippe**
**20180 Ain Diab / Casablanca (MA)**
- **SEBTI, Thami**
**1090 Bruxelles (BE)**
- **MERLOS, Romain**
**7070 Mignault (BE)**

(74) Representative: **Office Kirkpatrick**
**Avenue Wolfers, 32**
**1310 La Hulpe (BE)**

# (54) NEW DRY POWDER COMPOSITION OF TIOTROPIUM FOR INHALATION

(57) The present invention relates to a method of treating chronic lung diseases such as chronic obstructive pulmonary disease (COPD) by administering a therapeutically effective amount of inhaled tiotropium bromide in the form of a respirable dry powder composition administrable through a dry powder inhaler. The composition comprises tiotropium within a mixture of a coarse and a fine carrier, wherein the ratio of the components is such that the fine particle dose (FPD) of tiotropium is comprised between 1.7 µg and 3.7 µg for an administered dose of 12 µg or less.

EP 4 108 230 A1

## Description

Field of the invention

The present invention relates to a method of treating chronic lung diseases such as chronic obstructive pulmonary disease (COPD) by administering a therapeutically effective amount of inhaled tiotropium bromide.

Background of the invention

Local delivery of medications to the lung is highly desirable, especially in patients with specifically pulmonary diseases, such as cystic fibrosis, asthma, chronic pulmonary infections or lung cancer, but also, in some cases, to treat non pulmonary diseases, after absorption of the drugs in the respiratory tract and especially in alveoli. The principal advantages include reduced systemic side effects and higher dose levels of the applicable medication at the site of drug action. Unlike the oral route of drug administration, pulmonary inhalation is not subject to first pass metabolism. Drug inhalation enables a rapid and predictable onset of action, induces fewer side effects than does administration by other routes, and minimizes the risk of drug-drug interactions.

To achieve an optimal deposition of drug in the lungs, different formulation strategies can be applied according to the drug's physico-chemical properties. In most cases, the formulation strategies require the use of various solvents and/or excipients during the production step. These excipients have to be approved or "generally recognized as safe" (GRAS) substances for pulmonary drug delivery. Moreover, the residual solvents have to be below defined acceptable limits. Various guidelines for clinical toxicities and pharmacokinetic studies of these excipients have been proposed by the Food and Drug Administration (FDA) and the International Pharmaceutical Excipients Council.

Each drug formulation is preferably designed in relation with a corresponding inhaler to be administered efficiently by oral inhalation. The function of the inhaler is to deliver drug formulations reproducibly as solid or liquid particles suspended in the air (i.e., an aerosol of fine particles) that present a dae (aerodynamic equivalent diameter) between 0.5 and 5 $\mu$m. There are three types of inhalation devices: nebulizers, pressurized metered dose inhalers (pMDIs), and dry powder inhalers (DPIs). The operation of nebulizers requires a compressed gas (jet-nebulizers), a piezoelectric element to produce ultrasonic waves (ultrasonic nebulizers) or a vibrating mesh (vibrating mesh nebulizers) to generate solution liquid aerosols with sufficient fine particle fraction (FPF).

DPIs were developed to counter the problems of pMDIs. Namely, DPIs do not use propellants and do not need patient hand-breath coordination as the patient inhalational effort is responsible for DPI actuation. All DPIs have a dose metering mechanism or chamber, an aerosolization and a disaggregation mechanism, and an adaptor to direct the aerosol into the mouth. The solid state of formulations increases their long-term stability, although they are sensitive to relative humidity.

Stability is one of the most important factors which determines whether a compound or a mixture of compounds can be developed into a therapeutically useful pharmaceutical product. When mixing different ingredients in a pharmaceutical formulation, there exists the possibility of interactions taking place between the components.

Chronic obstructive pulmonary disease (COPD) is now one of the top three causes of death worldwide. More than 3 million people died of COPD in 2012 accounting for 6% for all deaths globally. COPD represents an important public health challenge that is both preventable and treatable. COPD is a major cause of morbidity and mortality throughout the world. Many people suffer from this disease for years and die prematurely from it or its complications. The COPD burden is projected to increase in coming years because of continued exposure to COPD risk factors and aging of the population.

COPD is a chronic, slowly progressive disorder characterized by airflow obstruction (associated with airways inflammation). COPD includes emphysema, chronic bronchitis, chronic airflow limitation, chronic airways obstruction, non-reversible obstructive airways disease, chronic obstructive airways disease and chronic obstructive lung disease. The clinical presentation of COPD can vary in severity from simple chronic bronchitis without disability to a severely disabled state with chronic respiratory failure. The diagnosis of COPD is usually suggested by symptoms but can only be established by quantitative measurements, preferably using spirometry (see Thorax, 1997; 52 suppl 5: S1-S32).

Anticholinergic bronchodilators have become standard care in treatment of COPD since the introduction of ipratropium bromide in the 1980s.

Tiotropium bromide (SPIRIVA® [Boehringer Ingelheim GmbH, Ingelheim am Rhein, Germany]) was the first long-acting muscarinic antagonist (LAMA) to be approved for maintenance treatment of chronic obstructive pulmonary disease (COPD). Tiotropium is used as an inhaled medication that helps to widen the airways (bronchodilating effect) for up to 24 hours, and is used to manage persistent symptoms of COPD. (P. Barnes; Exp. Opin. Invest. Drugs, 2001). It is available in two formulations: dry powder (18 $\mu$g once daily) delivered via the breath-actuated HANDIHALER®, and aqueous solution (5 $\mu$g, two puffs 2.5 $\mu$g once daily) delivered via the RESPIMAT® Soft Mist Inhaler.

More recently, similar products containing tiotropium produced by Teva (BRALTUS®) and Mylan (Sirkava®)

were granted a marketing authorization in 2016 and 2018 respectively and are available on the European (EU) market.

The chemical structure of tiotropium was first described in US 5610163. Different salts of this product (bromide, fluoride, chloride, iodine, sulphate, etc.) as well as different crystalline forms are known. For example, US 6,777,423 discloses a crystalline form of Tiotropium bromide monohydrate, US 6,608,055 and WO 2005/042527 disclose crystalline forms of anhydrous Tiotropium bromide, while IPCOM000143595D discloses a dichloromethane solvate of Tiotropium bromide. More crystalline forms are disclosed in EP1869035.

Attempts to develop inhalable dry powder to administer tiotropium salts for the treatment of COPD have been described. For example, WO2002/030389 discloses an inhalable powder containing 0.04 to 0.8% by weight of tiotropium. The inhalable powder contains tiotropium under its tiotropium bromide crystalline monohydrate form in admixture with a physiologically acceptable excipient. WO2010/037845 discloses inhalable particles comprising a stabilized amorphous form of tiotropium bromide with stabilizing agent obtained by the spray-drying technique wherein the particles are mixed with one or more coarse excipient having a mean particle size of 15 to 250 μm.

US 9,737,518 B2 discloses a respirable dry powder comprising dry particles that comprise sodium chloride, leucine, and tiotropium bromide obtained by spray-drying technique.

Tiotropium bromide is an expensive API and the delivery efficiency of the molecule to the lungs is currently low. Administration of an 18 μg dose as in Spiriva® currently leads to a drug delivery of only 10 μg from which around 3 μg are really deposited in the lung while around 7 μg are deposited in the oropharynx region, causing adverse effects.

The applicant has judged necessary to develop a new formulation of tiotropium using less API for a similar pharmacological effect and a better safety profile, thereby decreasing both the manufacturing costs and the side effects.

## Description of the invention

This invention relates to a respirable dry powder composition deliverable through a dry powder inhaler comprising tiotropium within a mixture of a coarse and a fine carrier, wherein the ratio of the components is such that the fine particle dose (FPD) of tiotropium reaching the pulmonary tract is comprised between 1.7 μg and 3.7 μg for an administered dose of 12 μg or less.

Preferably, the respirable dry powder composition of the invention is deliverable through a high resistance inhaler, wherein the resistance is comprised between 0.04 and 0.06 sqrt(kPA)/liters per minute.

Advantageously, in the respirable dry powder composition of the invention, the tiotropium represents between about 0.01% and 0.3% by weight of the composition.

Tiotropium means the free ammonium cation of tiotropium. The counter ion may be chloride, iodide, methane sulphonate, paratoluene sulphonate, or methyl sulphate. Of these, the bromide is preferred.

Any reference to tiotropium in the present application refers to the free ammonium form, in particular when weight data, like weight percentage is involved. The reference to tiotropium still implies the use of a salt and/or hydrate of tiotropium.

Any reference to tiotropium bromide should be taken as a reference to any amorphous or crystalline form of tiotropium bromide.

In the respirable dry powder composition of the invention, the tiotropium preferably has a particle size distribution, as measured by laser diffraction, of:

$d(50) \leq 5$ μm
$d(90) \leq 10$ μm.

This particle size distribution guaranties that the active ingredient reaches the respiratory tract during inhalation.

In the respirable dry powder composition of the invention, the coarse carrier preferably has an average particle size comprised between 40 μm and 200 μm, as measured by laser diffraction, allowing the desired good physical properties of the blend.

The fine carrier preferably has an average particle size, as measured by laser diffraction, comprised between 1 and 9 μm.

The weight % of the fine carrier can be comprised between 5 and 40% of the total weight of the excipients.

The fine and the coarse carriers can be a carbohydrate or a mixture of carbohydrates. The carbohydrates can be selected within the list comprising monosaccharides such as lactose, glucose, arabinose, disaccharides such as dextrose or polyalcohol such as sorbitol, mannitol, xylitol. The carbohydrate can be in an anhydrous or in a hydrated form. The coarse carrier and the fine carrier are preferably lactose.

In a particular embodiment, the respirable dry powder of the invention consists of tiotropium particle, preferably tiotropium bromide particles, coarse lactose particles and fine lactose particles.

In an advantageous embodiment, the respirable dry powder composition of the invention is inserted in containers for use with a dry powder inhaler, which can be unit-dose (single or multiple) containers like capsules, cartridges or

blisters, or bulk storage containers like multi-dose reservoirs, depending on the inhalation device. The invention also relates to a container for a dry powder inhaler comprising the respirable dry powder composition. In a preferred embodiment, the container is a unit-dose container. In a preferred embodiment, the unit-dose container is a capsule.

A capsule can be in any suitable material like gelatin or HPMC but is preferably a HPMC capsule since it is of vegetal origin. The capsule is preferably transparent to allow the user to verify the complete use of the content of the capsule. More preferably the capsule is a size 3 HPMC capsule.

A unit-dose container preferably comprises 12 μg of tiotropium or less, preferably 10 μg or less, still preferably 9 μg or less of tiotropium.

The invention also relates to a high resistance dry powder inhaler, wherein the resistance is comprised between 0.04 and 0.06 sqrt(kPA)/liters per minute, comprising at least one unit-dose container as described above.

As is well known by the person skilled in the art, the inhaler resistance is correlated to the respiratory flow required to produce a pressure drop of 4 kPa. A high resistance inhaler typically requires a respiratory flow of around 50 L/min or less.

Further to the low efficiency of the reference medicine Spiriva®, this product also has a limited stability and must be stored below 25 °C to maintain the formation of tiotropium's impurities at an acceptable level for several months.

It has been surprisingly observed that the composition of the invention is exceptionally stable and that the impurities level can be maintained at an acceptable level for several months, even in high temperature and high humidity conditions.

The stability is particularly achieved when storing the composition or the unit-dose container or the inhaler of the invention in a packaging comprising a desiccant, a water and oxygen barrier, and wherein the composition, the capsule or the inhaler is under an inert atmosphere.

The inert atmosphere preferably comprises less than 1% oxygen (volume oxygen: total gas volume). The powder can be as such in the packaging, or inserted in unit-dose container, or prefilled in a multi-dose container.

This packaging allows the total amount of impurities in the composition to remain below 1% after 12 months at 25 °C and 60% RH.

Preferably, the packaging comprises a first layer, being a bottle made of glass or of a plastic polymer and a second layer, being an aluminium layer.

In a particular, a suitable plastic polymer is high-density polyethylene (HDPE), for instance coloured in white, possibly with a push-fit cap in low-density poly-ethylene (LDPE) or popypropylene (LDPP).

A preferred desiccant is silica gel.

The invention also pertains to the use of the respirable dry powder of the invention whether alone, in a capsule or within a unit dose dry powder inhaler for the treatment or the prevention of a lung disease.

Preferably, the lung disease is chronic obstructive pulmonary disease (COPD).

Starting from a nominal dose (i.e. the administered dose) of 8.8 pg, the dry powder formulation of the present invention has a predicted oropharyngeal deposition lower than 7 μg of tiotropium, more preferably inferior to 6 μg and more preferably inferior to 5 μg when emitted from the high resistance dry powder inhaler. Hence, the dry powder formulation of the present invention has a low predicted risk of side effects (e.g. hypersensitivity, dry mouth, bronchospasm, constipation, intestinal obstruction etc.). The dry powder formulation has an efficiency to deliver a dose of tiotropium to the lung similar to that of the benchmark dry powder formulation Spiriva® with lower nominal dose filled into the capsule. Each capsule of Spiriva® contains 18 μg of tiotropium (the nominal / administered dose). The delivered dose (the dose that leaves the mouthpiece of the inhaler device , i.e. the dose delivered from the inhaler) is 10 μg. The Fine Particle Dose (FPD) (the dose deposited in the lungs or the respirable dose reaching the lungs) is around 2.8 μg ± 25%. The composition of the invention can lead to a similar FPD from a nominal dose and delivered doses of tiotropium of 12 μg or less of tiotropium and 10 μg or less respectively, for example from a nominal and delivered doses of 8.8 μg and 7.0 μg respectively. The pharmacological effect is thus ensured at lower production costs and side effects.

## Detailed description of the invention

A more complete appreciation of the disclosure can be obtained and understood from the following detailed description with the reference to the accompanying drawings, wherein:

Figure 1 is a plasma PK profile of Spiriva® compared to one composition of the invention.
Figure 2 is a graph showing the mean change in $FEV_1$ (forced expiratory volume in one second) from baseline over time from pre-dose to 24hours post dosing after administration of Spiriva® and one composition of the invention.

Definitions:

The term "dry powder" as used herein refers to compositions that comprise respirable dry particles.

The term "respirable" means suitable for inhalation or suitable for delivery to the lungs of a subject by inhalation.
The term "fine particle dose" (FPD) may be defined as the amount of drug of the inhaled product that is generally considered to be of a suitable aerodynamic size, i.e below 5 μm, able to reach the pulmonary tract, bind to the relevant receptors and produce the desired therapeutic effect in vivo. The test on aerodynamic assessment of fine particles is the main test for DPI formulations, aimed to assess the behaviour of particles in airflow, which mimics the in vivo conditions of deposition of particles in the human respiratory tract after inhalation of drugs. The European Pharmacopoeia (EP) - chapter 2.9.18 describes in details the way to perform this test as well as the way to calculate the FPD.
The term aerodynamic diameter means the diameter of a hypothetical sphere of density 1 g/cm$^3$ having the same terminal settling velocity in calm air as the particle in question, regardless of its geometric size, shape and true density.

$$d_{ae} = d\sqrt{\frac{\rho}{\rho_0 \chi}}$$

Where d is the geometric diameter of the particle, $\rho$ is the particle density, $\rho_0$ is the reference density and $\chi$ is a dynamic factor taking in account the particle shape.

Impactor provides a direct and reproducible measurement of both aerodynamic diameter and drug mass distribution, which are relevant to the deposition within lungs. The process is based on the impaction of the droplets/particles on different stages of the apparatus simulating particle deposition in the different sections of the respiratory tract.
The calculation by interpolation of the mass of active ingredient with an aerodynamic diameter of less than 5μm gives the FPD and it determines therefore the fraction of the active substance that can reach its lung target (FPF, Fine Particle Fraction = FPD expressed in function of the nominal dose).
A carrier is an important component of the DPI formulation. Since micronized drugs have poor flow properties, mixing the drug with a suitable carrier enhances the flow behaviour and thus improves dosing accuracy and consistency. Furthermore, the carrier facilitates the dispersion of the cohesive micronized drug during the inhalation event. Any change in the physiochemical properties of the carrier has the potential to alter the product performance and drug deposition in the lungs; therefore, selecting and optimizing the right carrier is an important step in developing a suitable DPI formulation.
Selection of a carrier is typically based on:

- Functionality: powder flow and dispersion during inhalation
- Stability: physicochemical stability, biocompatibility, biodegradability, and compatibility with the drug substance
- Safety: it must be inert and non-toxic to lung tissues.

Consequently, it has been stated that the efficiency of a DPI formulation is dependent on the carrier characteristics, and the selection of carrier is a determinant of the overall DPI performance. In case of DPI formulations, most of the micronized drug particles adhere to the surface of coarse carrier particles. During inhalation maneuvering, based on the velocity of inspired air flow, these drug particles are detached from the surface of the carrier particles. The larger carrier particles are deposited in the upper airways, while the small drug particles can reach the lower parts of the lungs. Detachment of drug from the carrier is based on the balance between inter-particulate adhesive forces and air flow velocity. The right cohesive and adhesive balance is, therefore, required between the drug and carrier to produce a stable formulation that at the same time allows easy separation during inhalation.
Typical carriers for inhalation are composed by coarse and fine excipients, the larger particles allowing good physical properties, the smaller one helping in mobilizing the active ingredient in the lung. In the present invention said coarse carrier has preferably d(50) between 40 μm and 200 μm, and the fine carrier has preferably d(50) of 9 μm or less. The preferred carrier is lactose but other carbohydrates may also be used such as monosaccharides (e.g. glucose, arabinose) disaccharides (e.g. dextrose), polyalcohol (e.g. sorbitol, mannitol, xylitol) or mixtures of these excipients with one another.
The dry powder formulation of the present invention preferably contains the tiotropium derivative in a micronized form. For the purpose of the present invention, "micronized" means an average particle size of 10 μm or less, and more preferably inferior to 7 μm when measured by laser diffraction for instance.
Compounds used in the examples are selected within the pharmaceutically acceptable purities and qualities.
Laser diffraction refers to a method where a beam of monochromatic light is directed at the sample particles

to be measured, which are, for instance, present in a suspension in a transparent liquid. When the light hits the particles, the light is diffracted or scattered from the particles. Detectors are used to measure the relative average intensity of the light scattered at various angles from the sample material, which then allows to calculate the particle size and size distribution.

The average particle size used here refers to the 50% value from the volume distribution (d(50)), i.e. the size in which 50 % of the sample is smaller and 50 % is larger, measured by a diffractometer using the wet or dry dispersion method, as described below.

The particle size determination of tiotropium was performed using a Malvern Mastersizer 2000 (Malvern Panalytical, UK) equipped with the Hydro S dispersion unit (Malvern Panalytical, UK), in accordance with the manufacturer's instructions, and with the following settings:

| | |
|---|---|
| Dispersing unit | Hydro S, wet dispersion |
| sample size range | 0.02 μm - 2000 μm |
| Ultrasonication rate | external Ultrasonicator |
| Measurement duration | 10 seconds |
| Dispersant medium | n-heptane |
| Dispersant refractive index | 1.39 |
| Particle refractive Index | 1.70 |
| Particle absorption | 0.05 |
| Stirrer rate: | 2500 ± 10 rpm |
| Obscuration level | 20 - 30% ± 2% |
| Analysis model | MIE, general purpose |

The particle size determination of coarse lactose was performed using a Malvern Mastersizer 2000 (Malvern Panalytical, UK) equipped with the dry dispersion unit (Malvern Panalytical, UK), in accordance with the manufacturer's instructions, and with the following settings:

| | |
|---|---|
| Dispersing unit | Scirocco, dry dispersion |
| sample size range | 0.02 μm - 2000 μm |
| Measurement duration | 20 seconds |
| Particle refractive Index | 1.52 |
| Particle absorption | 0.1 |
| Dispersion air pressure | 2 bars |
| Vibrating speed rate | 50% of the maximum speed |
| Obscuration level | 0.1 - 5% ± 2% |
| Analysis model | Mie, general purpose |

The particle size determination of fine lactose was performed using a Malvern Mastersizer 2000 (Malvern Panalytical, UK) equipped with the Hydro S dispersion unit (Malvern Panalytical, UK), in accordance with the manufacturer's instructions, and with the following settings:

| | |
|---|---|
| Dispersing unit | Hydro S, wet dispersion |
| sample size range | 0.02 μm - 2000 μm |
| Ultrasonication rate | external Ultrasonicator |
| Measurement duration | 12 seconds |
| Dispersant medium | ethanol |

(continued)

| | |
|---|---|
| Dispersant refractive index | 1.36 |
| Stirrer rate: | 800 ± 10 rpm |
| Obscuration level | 10 - 30% ± 2% |
| Analysis model | Fraunhofer, general purpose |

The Inhalable powder according to the invention are preferably filled into capsules with amount from 3 to 50 mg, preferably from 5 to 25mg.

The dry powder formulation of the present invention has a delivered dose of at least 60%, more preferably superior to 70% and more preferably superior to 75% of the nominal dose when emitted from the high resistance dry powder inhaler.

Capsules filled by the dry powder formulation of the present invention are contained in a receptacle. Preferably this receptacle is a HPDE bottle closed by a push-fit LDPP (low density polypropylene) cap with silica gel dehydrating capsule (about 2g) integrated into the cap. Each bottle preferably contains 30 capsules. The combination of the dry powder formulation filled into HPMC capsules contained in this receptacle prevents or slows down the physical transformation and the chemical degradation (e.g. hydrolysis, oxidation or any other degradation mechanism) of the active substance. It was surprinsingly observed that the physico-chemical transformation of tiotropium during storage (e.g. at 25°C/60% RH, 30°C/65% RH or 40°C/75% RH) is prevented. Hence, the physico-chemical stability and the aerodynamic stability of the dry powder formulation are maintained during at least 24 months and preferably superior to 30 months and more preferably superior to 36 months at 25°C/60% RH. Likewise, the physico-chemical stability and the aerodynamic stability of the dry powder formulation are maintained during 12 months and 6 months at 30°C/65% RH and 40°C/75% HR, respectively, which is not the case of the benchmark product.

In the present invention, total impurities (weight impurities: total weight tiotropium bromide) represent less than 5.0 wt% (preferably less than 4%, 3%, 2%, 1.5%, 1.0% of impurities) after 24 months under storage condition of 25°C/60% RH or after 12 months under Storage condition of 30°C/65% RH or after 6 months under storage condition of 40°C/75% RH.

The impurities are measured at different time and different condition according to the ICH Q1A(R2) either as such, or mixed with its carriers to evaluate long term stability, possibly within the capsule and within the packaging.

Conservation can be either at 25°C and under 60% of relative humidity (RH), or at higher temperatures and relative humidity, such as at 40°C and under 75% RH, to predict long term stability. Batches are rejected when the impurities are above 1% (wt impurities: weight tiotropium bromide).

The respirable dry powders of the invention is for use in the treatment or the prevention of a lung disease, like chronic bronchitis, emphysema, chronic obstructive pulmonary disease (COPD), asthma, airway hyper responsiveness, seasonal allergic allergy, bronchiectasis, cystic fibrosis, pulmonary parenchymal inflammatory conditions and the like, and/or for the treatment, reduction in incidence, or severity, and/or prevention of acute exacerbations of these chronic diseases caused by viral infections (e.g. influenza virus, parainfluenza virus, respiratory syncytial virus, rhinovirus, adenovirus, metapneumovirus, coxsackie virus, echo virus, corona virus, herpes virus, cytomegalovirus, and the like), bacterial infections (e.g. Streptococcus pneumoniae, Staphylococcus aureus, Burkholderis ssp., Streptococcus agalactiae, Haemophilus influenzae, Haemophilus parainfluenzae, Klebsiella pneumoniae, Escherichia coli, Pseudomonas aeruginosa, Moraxella catarrhalis, Chlamydophila pneumoniae, Mycoplasma pneumoniae, Legionella pneumophila, Serratia marcescens, Mycobacterium tuberculosis, Bordetella pertussis , and the like), fungal infections (e.g., Histoplasma capsulatum, Cryptococcus neoformans, Pneumocystis jiroveci, Coccidioides immitis , and the like) or parasitic infections (e.g., Toxoplasma gondii, Strongyloides stercoralis , and the like), or environmental allergens and irritants (e.g., aeroallergens, including pollen and cat dander, airborne particulates, and the like). In a preferred embodiment, the pulmonary disease is chronic bronchitis, emphysema, or chronic obstructive pulmonary disease. If desired, the respirable dry powders and respirable dry particles can be administered orally.

In a preferred embodiment, the pulmonary disease is asthma.

In another preferred embodiment, the pulmonary disease is COPD.

Tiotropium bromide is chemically described as (1α, 2β, 4 β, 5α, 7β)-7-[(Hydroxydi-2-thienylacetyl)oxy]-9, 9-dimethyl-3-oxa-9-azoniatricyclo[3.3.1.02,4] nonane bromide monohydrate. It is a synthetic, non-chiral compound. Tiotropium bromide is a quaternary ammonium salt and there is no other ionisable functional group on the molecule.

Tiotropium bromide is a white or yellowish white powder. It is sparingly soluble in water and soluble in methanol. The active substance is not optically active.

The structural formula of tiotropium bromide is C19H22BrNO4S2. Molecular Weight 472.4 g/mol. The chemical formula of Tiotropium bromide is as follows:

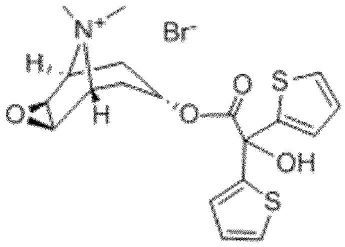

In the examples below, "tiotropium" means the free ammonium cation, while tiotropium bromide refers to the bromide salt of tiotropium.

The weight of tiotropium bromide is to be understood as its equivalent weight in anhydrous form.

Moreover, the weight of the tiotropium species is expressed in the literature as weight of tiotropium bromide or as weight of tiotropium, which is about 16% lower considering the absence of the bromide anion.

Example 1 - Compositions according to the invention vs SPIRIVA®

As a benchmark product, Spiriva® Handihaler® 18 µg has been studied and evaluated in numerous different studies. The mean in vitro pulmonary deposition (estimated by the assessment of the FPD, i.e. the mass of particles with aerodynamic diameter under 5 µm fraction described in the bibliography for this product) is about 2.1 - 3.5 µg of tiotropium.

As described below, the fine particle fraction denotes the fraction of the dry powder formulation able to reach the pulmonary tract, bind to the relevant receptors and produce the desired therapeutic effect.

Several compositions according to the invention have been prepared in order to match the FPD of the reference product. Their content is summarized in Table 1, 2 and 3.

The impaction test were performed using the apparatus E as described in the European Pharmacopoeia (chapter 2.9.18). The capsule based DPI is a high resistance DPI i.e., DPI has a resistance of about 0.05 sqrt(kPA)/liters per minute (±15%). The quantification of the deposited tiotropium on the different stages was determined by HPLC analysis. The results as shown in Table 1, 2 and 3.

Example 1a/

Coarse carrier: Anhydrous Lactose d(50): 150 µm
Fine carrier: Micronized Lactose Monohydrate d(50): 5 µm

Table 1. *: the ratio means % of coarse carrier within excipients and % of fine carrier within excipients.

| | Composition | 1 | 2 | 3 | 4 | 5 | 6 | 7 | 8 |
|---|---|---|---|---|---|---|---|---|---|
| Tiotropium free ammonium cation | amount (µg) | 8.8 | 8.8 | 8.8 | 8.8 | 8.8 | 10.0 | 10.0 | 12.0 |
| | w/wt (%) | 0.04 | 0.04 | 0.04 | 0.04 | 0.04 | 0.04 | 0.04 | 0.048 |
| | Coarse : fine lactose ratio | 75/25 | 100/0 | 95/5 | 85/15 | 65/35 | 75/25 | 85/15 | 75/25 |
| | FPD (µg) | 2.5 | 1.4 | 1.7 | 3.3 | 2.1 | 2.8 | 2.7 | 3.4 |

Table 1 shows that *in vitro* lung deposition in the same range as Spiriva® (2.1-3.5 µg) is obtained for the composition 1, 4, 5, 6, 7 and 8 with coarse/fine ratio ranged between 65/35 and 85/15 %(w/w lactoses); the coarser excipient having an average particle size of 150 µm.

Example 1b/

Coarse carrier: Lactose Monohydrate d(50): 85 µm
Fine carrier: Micronized Lactose Monohydrate d(50): 5 µm

Table 2. *: the ratio means % of coarse carrier within excipients and % of fine carrier within excipients.

| Composition | | 1 | 2 | 3 |
|---|---|---|---|---|
| Tiotropium free | amount | | | |

(continued)

| Composition | 1 | 2 | 3 |
|---|---|---|---|
| ammonium (μg)<br>cation w/wt (%) | 10<br>0.04 | 10<br>0.04 | 12<br>0.048 |
| Coarse / fine lactose ratio* | 75/25 | 85/15 | 90/10 |
| FPD (μg) | 2.6 | 2.7 | 3.2 |

Table 2 shows that *in vitro* lung deposition in the same range as Spiriva® (2.1-3.5 μg) is obtained for the composition 1, 2 and 3 with coarse/fine ratio ranged between 75/25 and 90/10 % (w/w lactoses; the coarser excipient having an average particle size of 85 μm.

Example 1c/

Coarse carrier: Lactose Monohydrate d(50): 60 μm
Fine carrier: Micronized Lactose Monohydrate d(50): 5 μm

Table 3. *: the ratio means % of coarse carrier within excipients and % of fine carrier within excipients.

| Composition | 1 |
|---|---|
| Tiotropium free ammonium cation amount (μg)<br>w/wt (%) | 8.8<br>0.04 |
| Coarse / fine lactose ratio* | 75/25 |
| FPD (μg) | 2.6 |

Table 3 shows that *in vitro* lung deposition in the same range as Spiriva® (2.1-3.5 μg) is obtained for the composition 1 with coarse/fine ratio 75/25 % (w/w lactoses; the coarser excipient having an average particle size of 60 μm.

Example 2 - Evaluation of the Stability of the new composition

The tiotropium content and total impurities content of the composition 1 of example 1a/ were evaluated by HPLC analysis at different storage condition 25°C and 60% RH, 30°C and 65% RH and 40°C and 75% RH. Results obtained indicate acceptable stability up to 6 months at 40°C and 75% RH (Table 6), i.e. under stringent/accelerated storage conditions, 12 months at 30°C and 65% RH (Table 5), and 18 month at 25°C and 60% RH (Table 4).

In comparison, the benchmark product, Spiriva®, has to be stored below 25°C since total impurities are out of specifications at 12 months 30°/65% RH (data not shown, derived from public domain ressources, e.g. summary of product characteristics (SmPC) available at:https://www.medicines.org.uk/emc/product/1693/smpc#SHELF LIFE (accessed 10 June 2021) and at 40°/75% RH (table 7).

Table 4.

| Storage condition 25°C/60% RH | T0 | T3 | T6 | T9 | T12 | T18 |
|---|---|---|---|---|---|---|
| Tiotropium content (%) | 97.1 | 98.0 | 99.1 | 98.8 | 97.0 | 98.4 |
| Total Impurities (%) | 0.1 | 0.1 | 0.1 | 0.1 | 0.1 | 0.1 |

Table 5.

| Storage condition 30°C/65% RH | T0 | T3 | T6 | T9 | T12 |
|---|---|---|---|---|---|
| Tiotropium content (%) | 97.1 | 96.1 | 98.9 | 100.9 | 97.2 |
| Total Impurities (%) | 0.1 | 0.1 | 0.1 | 0.1 | 0.1 |

Table 6.

| Storage condition 40°C/75% RH | T0 | T3 | T6 |
|---|---|---|---|
| Tiotropium content (%) | 97.1 | 96.7 | 97.4 |
| Total Impurities (%) | 0.1 | 0.1 | 0.2 |

Table 7.

| Spiriva® Batch 307333 | T0 | 25°C/ 60% RH | | | | 40°C / 75% RH | |
|---|---|---|---|---|---|---|---|
| | | T03 | T06 | T09 | T12 | T03 | T06 |
| Tiotropium content (%) | 99.3 | - | 95.1 | - | 98.3 | 92.3 | 85.8 |
| Total impurities (%) | - | - | 0.5 | - | 0.9 | 2.5 | 2.7 |

Example 3 - Pharmacokinetics

Pharmacokinetics studies were performed with the compositions 1 of example 1a/ administered using a high resistance device (0.04 and 0.06 sqrt(kPA)/liters per minute). The reference Spiriva® Handihaler® (18 $\mu$g tiotropium) was used as comparative treatment. The evolution of plasma concentration over time for both formulations are given in figure 1.

In details, an HPMC capsule #3 (transparent body and cap, hypromellose) is filled with 22 mg of composition 1.

In this PK study, a single dose was administered to 36 subjects in fasting conditions, confined at least 12h prior to drug administration and 24h post administration. Tiotropium was determined in all relevant plasma samples using a validated chromatographic method (LC coupled on-line MS-MS) according to Good Laboratory Practises Procedures (EMEA/CHMP/EWP/192217/2009 Rev. 1 Corr*, 21 July 2011). The standard pharmacokinetics parameters $C_{max}$, AUCo-t, $AUC_{0-30min}$ were determined from the plasma concentrations of tiotropium.

$C_{max}$ is defined as the maximal plasma concentration observed after the administration of a study treatment. This value was taken directly from the plasma concentration time curve. If not unique, the first maximum was used. This parameter is expressed in pg/mL.

AUCo-t is defined as the area under the plasma concentration curve from administration to the last measurable concentration. The AUC from time zero to the last quantifiable measurement was estimated by the validated software using the lin up/log down method. The lin up/log down method is a computation method where all ascending phases are computed in a linear manner and all descending phases are computed based on their log values. This parameter is expressed in pg/mL.min.

$AUC_{0-30min}$ is defined as the partial area under the plasma concentration curve from administration to the defined end time (30 minutes) . This partial AUC was estimated by the validated software using the lin up/log down method. The lin up/log down method is a computation method where all ascending phases are computed in a linear manner and all descending phases are computed based on their log values. This parameter will be expressed in pg/mL.min.

In the present study, only comparison of parameters predictive of the lung deposition ($C_{max}$, $AUC_{0-30}$, AUCo-t) is relevant to evaluate the similarity of the efficacy of these products and comparison of the total systemic exposure should only be used for safety concerns.

The pharmacokinetic parameters were calculated with a non-compartmental analysis (NCA) model of the validated software as required by the guidance. The actual time of sampling was used for the estimation of the pharmacokinetic parameters.

The mean plasma concentration profiles for composition 1 of example 1a and Spiriva® are shown in figure 1.

The results of the PK analysis are shown in the table 8.

Table 8.

| | composition 1 (example 1a) | Spiriva® |
|---|---|---|
| $C_{max}$ (pg/mL) | 11.59 $\pm$ 8.33 | 11.53 $\pm$ 10.18 |
| $AUC_{0-30}$ (pg/ml.min) | 179.34 $\pm$ 113.19 | 191.15 $\pm$ 153.60 |
| $AUC_{0-t}$ (pg/ml.min) | 1144.55 $\pm$ 455.50 | 1532.22 $\pm$ 602.22 |

Exposure profiles obtained with composition 1 of the example 1a is very similar to exposures obtained with Spiriva® despite a much lower amount of tiotropium present in the capsules, demonstrating the superiority of the formulation of the compositions according to the present invention. The clinical data demonstrates the ability to reach lung dose of Spiriva® with a significantly lower nominal dose. The $C_{max}$ obtained after the inhalation of the composition 1, 11.59 pg/mL, is similar to that obtained after the Spiriva® inhalation, which was 11.53 pg/mL. The $AUC_{0-30}$ obtained with the composition 1 was 179.3 pg/mL.min, which matched that of Spiriva® (191.1 pg/mL.min). This means that the pharmacological effect of composition 1 is expected be the same that of Spiriva® for an administered dose of API as low as half the amount of tiotropium used in Spiriva®. This API being particularly expensive, a relevant economic benefit is therefore expected.
The AUCo-t of composition 1 was 1144.6 pg/mL.min which was about 75% of the AUCo-t obtained with Spiriva®, 1532.2 pg/mL.min. This means that the systemic exposure of composition 1 was lower that of Spiriva®. This means that the safety profile of the composition 1 (example 1a) is expected to be better than that of Spiriva®.

Example 4 - Pharmacodynamics

In a phase II study, subjects were randomized to receive single doses of the reference product Spiriva® 18 μg, taken by inhalation via the Handihaler® and the test product composition 1 of example 1a taken by inhalation via a high resistance inhaler (0.05 sqrt(kPA)/liters per minute ±15%).
$FEV_1$, the forced expiratory volume in 1 second, refers to the volume of air that an individual can exhale during a forced breath in 1 second.
This study was a multi-centre, single-dose, randomized, active-controlled, partially-blinded, dose-ranging, three-way crossover study in patients with COPD, with an age range 44-80 years. COPD patients with a forced expiratory volume in 1 second ($FEV_1$) 30-80% of predicted normal and a postbronchodilator $FEV_1$/forced vital capacity (FVC) ratio <0.7 at screening were recruited. Patients were required to demonstrate ≥100 ml and at least 12% of reversibility in $FEV_1$ to maximum 400 μg of inhaled salbutamol.
Eligible subjects participated in five treatment visits, separated by washout periods of at least 3 days and maximum 14 days between each period.
The primary objective of this study was to assess the non-inferiority between the composition 1 of the example 1a and Spiriva® 18μg Handihaler® by measurement of the bronchodilating effect.
As required by the Guideline on clinical investigation of medicinal products in the treatment of COPD (EMA/CHMP/483572/2012 -corr1), through $FEV_1$ response defined as change in $FEV_1$ from baseline to $FEV_1$ 24h post-dose measurement was the primary endpoint.
Sixty-six subjects between 44 and 80 years old entered the study and were randomized and took in a cross-over way at least one of the two following treatments; Composition 1 of the example 1a, and Spiriva® Handihaler® 18 μg. Three subjects prematurely dropped out the study and 63 subjects completed the three periods. The primary efficacy criterion was the trough $FEV_1$ response defined as change in $FEV_1$ from baseline to $FEV_1$ 24h post-dose measurement. The null hypothesis of inferiority of composition 1 against Spiriva® Handihaler® 18 μg was rejected. Indeed the contrast of trough $FEV_1$ between composition 1 and Spiriva® Handihaler® 18 μg was 0.015 [90% CI= - 0.041; 0.070] for a non-inferiority threshold of -0.100 (p=0.596). The non-inferiority of composition against Spiriva® Handihaler® 18 μg was therefore confirmed (Table 9 and Figure 2).
In addition, no statistical significant difference was observed between composition 1 of example 1a and Spiriva® Handihaler® 18 μg regarding all other endpoints; baseline-adjusted AUC of $FEV_1$ from 0 to 24h post-dose and partial baseline-adjusted AUC from 0 to 4h, from 4 to 8h, from 8 to 12h and 12 to 24h post-dose, maximal value of $FEV_1$, Tmax of $FEV_1$, maximal value of peak expiratory flow (PEF), change in PEF at 24h post-dose from baseline, baseline-adjusted AUC PEF from 0 to 24h post-dose, baseline-adjusted AUC of $FEV_1$ from 0 to 24h post-dose; maximum value of $FEV_1$, baseline-adjusted AUC forced vital capacity (FVC) from 0 to 24h post-dose, maximum value of FVC and change in FVC at 24h post-dose from baseline.

Table 9.

| Variable | Modality | Composition 1 - 8.8 μg DPI (N=63) | Spiriva® Handihaler 18 μg (N=63) |
|---|---|---|---|
| $FEV_1$ (L/sec) at period-specific baseline | M (Missing) | 63 (0) | 63 (0) |
| | Mean (± Std) | 1.280 (±0.360) | 1.286 (±0.373) |
| | Median | 1.280 | 1.290 |
| | Q1; Q3 | 1.020; 1.530 | 1.020; 1.520 |
| | Min; Max | 0.500; 2.110 | 0.460; 2.330 |

(continued)

| Variable | Modality | Composition 1 - 8.8 μg DPI (N=63) | Spiriva® Handihaler 18 μg (N=63) |
|---|---|---|---|
| $FEV_1$ (L/sec) at 24h post-dose | M (Missing)<br>Mean (± Std)<br>Median<br>Q1; Q3<br>Min; Max | 62 (1)<br>1.374 (±0.412)<br>1.370<br>1.100; 1.630<br>0.390; 2.540 | 63 (0)<br>1.360 (±0.396)<br>1.340<br>1.100; 1.630<br>0.410; 2.270 |
| Through $FEV_1$ (L/sec) response | M (Missing)<br>Mean (± Std)<br>Median<br>Q1; Q3<br>Min; Max | 62 (1)<br>0.091 (±0.169)<br>0.085<br>-0.030; 0.180<br>-0.280; 0.530 | 63 (0)<br>0.075 (±0.193)<br>0.080<br>-0.030; 0.190<br>-0.410; 0.620 |
| **Estimates by products** | | | |
| - Composition 1 of example 1a | | LS means +/- SE<br>95% CI | 0.090 +/-0.0223<br>[0.045; 0.134] |
| - Spiriva® Handihaler 18 μg | | LS means +/- SE<br>95% CI | 0.075 +/-0.0222<br>[0.031; 0.118] |
| **Contrasts between products** | | | |
| - between Composition 1 vs. Spiriva® Handihaler 18 μg | | Estimate of the difference | 0.015 |
| | | 95% CI | [-0.041; 0.070] |
| | | P-value | 0.596 (NS) |
| | | Is the lower bound of the 95% CI higher than - 100 mL? | Yes |

This clinical study resulted in a similar lung function improvement as demonstrated by the similar improvements in FEV1 at the 24h timepoint between both products.

## Claims

1. Respirable dry powder composition administrable through a dry powder inhaler comprising tiotropium within a mixture of a coarse and a fine carrier, wherein the ratio of the components is such that the fine particle dose (FPD) of tiotropium is comprised between 1.7 μg and 3.7 μg for an administered dose of 12 μg or less.

2. Respirable dry powder composition according to claim 1, administrable through a high resistance inhaler, wherein the resistance is comprised between 0.04 and 0.06 sqrt(kPA)/liters per minute.

3. Respirable dry powder composition according to one of claims 1 or 2, wherein the tiotropium represents between about 0.01% and 0.3% by weight of the composition.

4. Respirable dry powder composition according to one of claims 1 to 3, wherein the tiotropium has a particle size distribution measured by laser diffraction of:

   $d(50) \leq 5$ μm
   $d(90) \leq 10$ μm

5. Respirable dry powder composition according to one of claim 1 to 4, wherein the coarse carrier has an average particle size comprised between 40 μm and 200 μm and the average particle size of the fine carrier is comprised between 1 and 9 μm, as measured by laser diffraction.

6. Respirable dry powder composition according to anyone of claim 1 to 5, wherein the fine and the coarse carriers

are a carbohydrate or a mixture of carbohydrates, preferably selected within the list comprising monosaccharides such as lactose, glucose, arabinose), disaccharides such as dextrose or polyalcohol such as sorbitol, mannitol, xylitol.

**7.** Respirable dry powder composition according to anyone of claim 1 to 6, wherein the weight % of the fine carrier is comprised between 5 and 40% of the total weight of the excipients.

**8.** Unit-dose container for a dry powder inhaler comprising the respirable dry powder composition of any of claim 1 to 7.

**9.** Unit-dose container according to claim 8, comprising less than 12 μg, preferably less than 10 μg, preferably less than 9 μg of tiotropium.

**10.** Unit-dose container according to any one of claims 8 or 9, wherein the unit-dose container is a capsule, preferably a HPMC capsule.

**11.** High resistance dry powder inhaler, wherein the resistance is comprised between 0.04 and 0.06 sqrt(kPA)/liters per minute, comprising at least one unit-dose container according to one of claims 8 to 10.

**12.** Packaging for the respirable dry powder composition according to one of claims 1 to 7 or the unit-dose container according to one of claims 8 to 10 or the high resistance dry powder inhaler of claim 11, comprising a desiccant, a water and oxygen barrier, wherein the composition, the container or the inhaler is under an inert atmosphere.

**13.** Packaging according to claim 12, wherein the total amount of impurities of tiotropium in the composition is below 1% after 12 months at 30°C and 65% RH.

**14.** Packaging according to one of claim 12 and 13, comprising a first layer, being a bottle made of glass or of a plastic polymer and a second layer, being an aluminium layer, wherein the bottle preferably is a HPDE bottle closed by a push-fit LDPP cap.

**15.** Respirable dry powder composition according to one of claim 1 to 7 for use in the treatment or the prevention of a lung disease and wherein the lung disease is preferably chronic obstructive pulmonary disease (COPD).

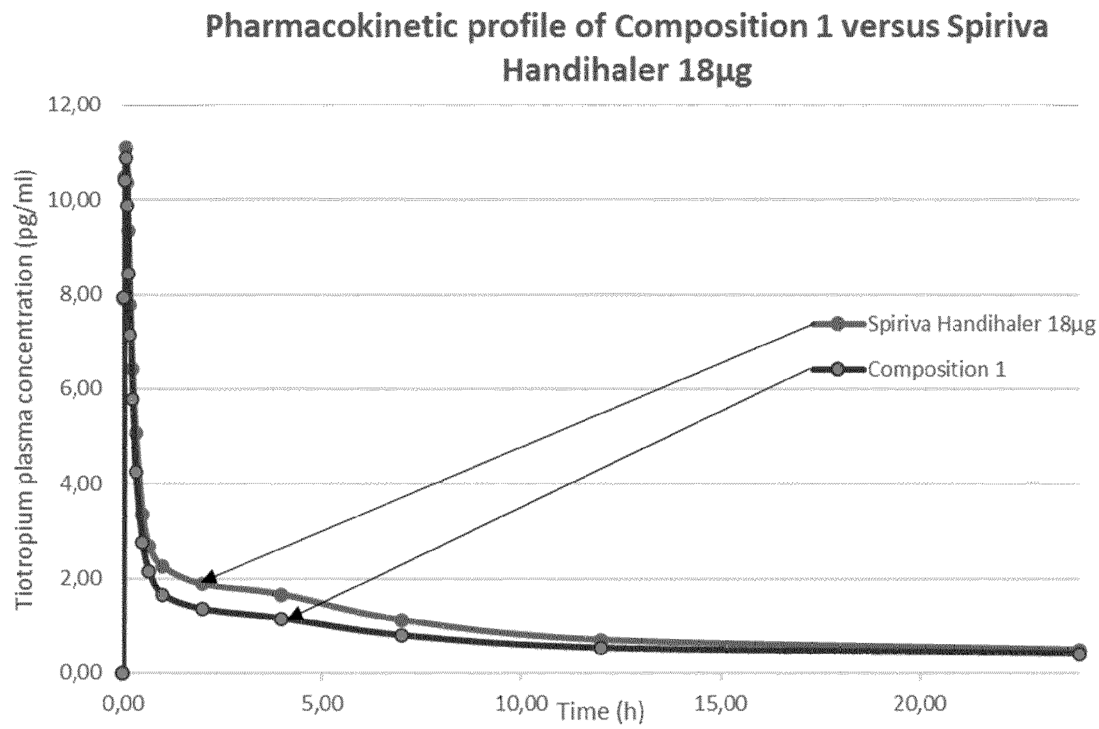


Figure 1.

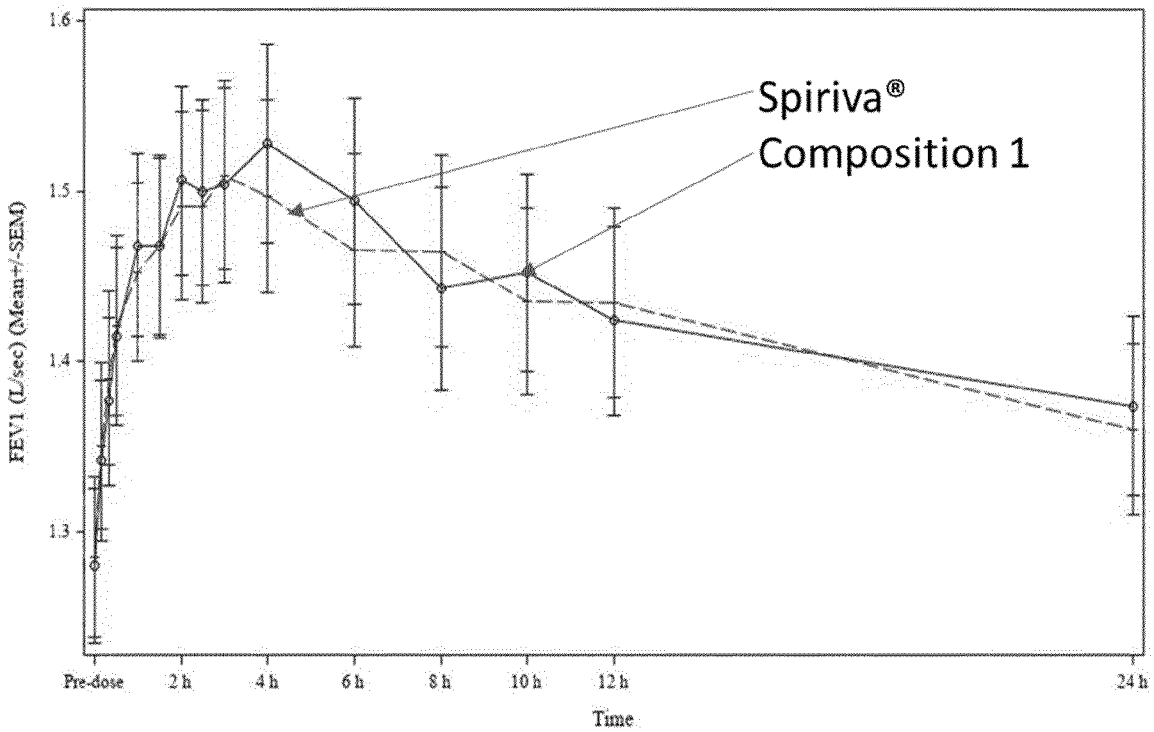


Figure 2.

Europäisches Patentamt
European Patent Office
Office européen des brevets

# EUROPEAN SEARCH REPORT

Application Number
EP 21 18 1419

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 101 422 457 B (JIANGSU CHIA TAI TIANQING PHAR) 26 January 2011 (2011-01-26) * paragraphs [0027] - [0028]; claims 1-17; examples 1-9 * ----- | 1-15 | INV. A61K9/00 A61K31/439 A61P11/00 A61K9/16 |
| X,D | EP 2 172 190 A1 (LICONSA LABORATORIOS SA [ES]) 7 April 2010 (2010-04-07) * paragraph [0082]; example 11 * ----- | 1-15 | |
| X | WO 2014/165303 A1 (PULMATRIX INC [US]) 9 October 2014 (2014-10-09) * paragraphs [0077], [0078]; claims 16,75-77 * ----- | 1-15 | |
| X | EP 2 821 062 A1 (ARVEN ILAC SANAYI VE TICARET AS [TR]) 7 January 2015 (2015-01-07) * paragraph [0036]; claim 1 * ----- | 1-15 | |
| | | | TECHNICAL FIELDS SEARCHED (IPC) |
| | | | A61K A61P |

1 The present search report has been drawn up for all claims

| Place of search | Date of completion of the search | Examiner |
|---|---|---|
| The Hague | 7 December 2021 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 18 1419

This annex lists the patent family members relating to the patent documents cited in the above-mentioned European search report. The members are as contained in the European Patent Office EDP file on
The European Patent Office is in no way liable for these particulars which are merely given for the purpose of information.

07-12-2021

| Patent document cited in search report | Publication date | Patent family member(s) | Publication date |
|---|---|---|---|
| CN 101422457 B | 26-01-2011 | NONE | |
| EP 2172190 A1 | 07-04-2010 | AR 073528 A1 | 10-11-2010 |
| | | AU 2009299801 A1 | 08-04-2010 |
| | | BR PI0920706 A2 | 29-12-2015 |
| | | CA 2737399 A1 | 08-04-2010 |
| | | CL 2011000696 A1 | 29-07-2011 |
| | | CN 102170861 A | 31-08-2011 |
| | | CO 6361899 A2 | 20-01-2012 |
| | | CR 20110227 A | 24-06-2011 |
| | | CY 1121090 T1 | 11-12-2019 |
| | | DK 2349204 T3 | 16-07-2018 |
| | | DO P2011000091 A | 30-04-2011 |
| | | EC SP11010925 A | 29-07-2011 |
| | | EP 2172190 A1 | 07-04-2010 |
| | | EP 2349204 A1 | 03-08-2011 |
| | | ES 2675857 T3 | 13-07-2018 |
| | | HN 2011000864 A | 09-02-2015 |
| | | HR P20181009 T1 | 24-08-2018 |
| | | HU E038208 T2 | 29-10-2018 |
| | | JP 5622730 B2 | 12-11-2014 |
| | | JP 2012504580 A | 23-02-2012 |
| | | KR 20110081265 A | 13-07-2011 |
| | | LT 2349204 T | 10-10-2018 |
| | | MA 34560 B1 | 02-10-2013 |
| | | MY 159172 A | 30-12-2016 |
| | | NI 201100066 A | 05-01-2012 |
| | | NZ 591923 A | 24-02-2012 |
| | | PE 20110850 A1 | 25-11-2011 |
| | | PE 20142435 A1 | 11-01-2015 |
| | | PL 2349204 T3 | 28-09-2018 |
| | | PT 2349204 T | 06-07-2018 |
| | | RU 2011117256 A | 10-11-2012 |
| | | RU 2014150986 A | 10-06-2015 |
| | | SI 2349204 T1 | 31-08-2018 |
| | | SV 2011003852 A | 12-04-2011 |
| | | TR 201808269 T4 | 23-07-2018 |
| | | TW 201018468 A | 16-05-2010 |
| | | UA 100777 C2 | 25-01-2013 |
| | | US 2011311618 A1 | 22-12-2011 |
| | | UY 32160 A | 30-04-2010 |
| | | WO 2010037845 A1 | 08-04-2010 |
| | | ZA 201101735 B | 30-05-2012 |
| WO 2014165303 A1 | 09-10-2014 | AU 2014248455 A1 | 15-10-2015 |
| | | BR 112015025160 A2 | 18-07-2017 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 21 18 1419

This annex lists the patent family members relating to the patent documents cited in the above-mentioned European search report. The members are as contained in the European Patent Office EDP file on
The European Patent Office is in no way liable for these particulars which are merely given for the purpose of information.

07-12-2021

| Patent document cited in search report | Publication date | Patent family member(s) | Publication date |
|---|---|---|---|
| | | CA 2907566 A1 | 09-10-2014 |
| | | CN 105324106 A | 10-02-2016 |
| | | EP 2981247 A1 | 10-02-2016 |
| | | HK 1221175 A1 | 26-05-2017 |
| | | JP 6473738 B2 | 20-02-2019 |
| | | JP 2016515576 A | 30-05-2016 |
| | | KR 20150135328 A | 02-12-2015 |
| | | RU 2015146871 A | 10-05-2017 |
| | | US 2016120855 A1 | 05-05-2016 |
| | | US 2017312258 A1 | 02-11-2017 |
| | | WO 2014165303 A1 | 09-10-2014 |
| EP 2821062 A1 | 07-01-2015 | EP 2821061 A1 | 07-01-2015 |
| | | EP 2821062 A1 | 07-01-2015 |
| | | ES 2662847 T3 | 10-04-2018 |
| | | PL 2821061 T3 | 30-05-2018 |
| | | PT 2821061 T | 13-03-2018 |
| | | TR 201802969 T4 | 21-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5610163 A
- US 6777423 B
- US 6608055 B
- WO 2005042527 A
- EP 1869035 A
- WO 2002030389 A
- WO 2010037845 A
- US 9737518 B2


### Non-patent literature cited in the description


- *Thorax,* 1997, vol. 52 (5), S1-S32
- **P. BARNES.** *Exp. Opin. Invest. Drugs,* 2001
- *Good Laboratory Practises Procedures (EMEA/CHMP/EWP/192217/2009 Rev. 1 Corr*,* 21 July 2011